# EUROPEAN PATENT SPECIFICATION

(11) **EP 1 708 711 B1**
(45) Date of publication and mention of the grant of the patent: **17.03.2010**
(21) Application number: 05708123.4
(22) Date of filing: 19.01.2005
(51) Int. Cl.: A61K 31/51, A23L 1/302, A61P 3/04, A61P 3/10, A61P 9/10

(54) **COMPOSITIONS USEFUL ESPECIALLY FOR TREATMENT OR PREVENTION OF METABOLIC SYNDROME**
BESONDERS NÜTZLICHE ZUSAMMENSETZUNGEN FÜR DIE BEHANDLUNG ODER PRÄVENTION DES METABOLISCHEN SYNDROMS
COMPOSITIONS PARTICULIEREMENT UTILES AU TRAITEMENT OU A LA PREVENTION DU SYNDROME METABOLIQUE

(30) Priority: 30.01.2004 FI 20040136; 14.06.2004 US 578896 P
(43) Date of publication of application: 11.10.2006
(73) Proprietor: Faron Pharmaceuticals OY, 20520 Turku (FI)
(72) Inventor: JALKANEN, Sirpa, FI-20760 Piispanristi (FI); SALMI, Marko, FI-20900 Turku (FI); JALKANEN, Markku, FI-20760 Piispanristi (FI)
(74) Representative: Öhman, Ann-Marie
(86) International application number: PCT/FI2005/000035
(87) International publication number: WO 2005/072738

(56) References cited:
- WO-A1-02/38153
- WO-A1-03/006003
- WO-A2-02/02090
- WO-A2-02/02541
- US-A- 4 558 050
- DATABASE MEDLINE [Online] YU P.H. ET AL: 'Aminoguanidine inhibits semicarbazide-sensitive amine oxidase activity; implications for advanced glycation and diabetic complications', XP002987373 Database accession no. (9389414) & DIABETOLOGIA vol. 40, no. 11, pages 1243 - 1250
- DATABASE MEDLINE [Online] BABAEI-JADIDI ROYA ET AL: 'Prevention of incipient diabetic nephropathy by high-dose thiamine and benfotiamine', XP002987374 Database accession no. (12882930) & DIABETES vol. 52, no. 8, August 2003, pages 2110 - 2120
- DATABASE MEDLINE [Online] AVENA R. ET AL: 'Thiamine (Vitamine B1) protects against glucose- and insulin-mediated profileration of human infragenicular arterial smooth muscle cells', XP002987375 Database accession no. (10629262) & ANNALS OF VASCULAR SURGERY vol. 14, no. 1, January 2000, pages 37 - 43
- DATABASE MEDLINE [Online] BAKKER S.J. ET AL: 'The association of dietary fibres with glucose tolerance is partly explained by concomitant intake of thiamine: The Hoorn Study', XP002987376 Database accession no. (9794103) & DIABETOLOGIA vol. 41, no. 10, October 1998, pages 1168 - 1175
- DATABASE WPI Week 197919, Derwent Publications Ltd., London, GB; Class A61, AN 1979-36101B, XP002987377 & JP 54 041 883 A (TAKEDA CHEM IND LTD) 03 April 1979
- DATABASE MEDLINE [Online] OBRENOVICH M.E. ET AL: 'Vitamin B1 blocks damage caused by hyperglcemia', XP002987378 Database accession no. (12844520) & SCIENCE OF AGIN KNOWLEDGDE ENVIRONMENT SAGE KE vol. 2003, no. 10, page PE6

## Description

### FIELD OF THE INVENTION

The present invention relates to the use of inhibitors of semicarbazide-sensitive amine oxidases (SSAO) for treatment or prevention of metabolic syndrome and diseases or conditions resulting therefrom. The invention also concerns the use of vitamin B1 or its metabolites as SSAO-inhibitors.

### BACKGROUND OF THE INVENTION

VAP-1 is a human endothelial cell adhesion molecule that has several unique properties that distinguish it from the other inflammation-related adhesion molecules. One of the most interesting features of VAP-1 is a catalytic extracellular domain which contains a monoamine oxidase activity (Smith, D. J., et al., J. Exp. Med 188:17-27 (1998)).

The cloning and sequencing of the human VAP-1 cDNA revealed that it encodes a transmembrane protein with homology to a class of enzymes called the copper-containing amine oxidases (E.C. 1.4.3.6). Enzyme assays have shown that VAP-1 possesses a monoamine oxidase (MAO) activity which is present in the extracellular domain of the protein (Smith, D. J., et al., J. Exp. Med. 188:17-27 (1998)). Thus, VAP-1 is an ecto-enzyme. Analysis of the VAP-1 MAO activity showed that VAP-1 belongs to the class of membrane-bound MAO's termed semicarbazide-sensitive amine oxidases (SSAO). These are distinguished from the widely distributed mitochondrial MAO-A and B flavoproteins by amino acid sequence, cofactor, substrate specificity and sensitivity to certain inhibitors. However, certain substrates and inhibitors are common to both SSAO and MAO activities. The mammalian SSAO's can metabolize various monoamines produced endogenously or absorbed as dietary or xenobiotic substances. They act principally on primary aliphatic or aromatic monoamines such as methylamine or benzylamine (Lyles G. A., Int. J. Biochem. Cell Biol, 28:259-274 (1996)). Thus, VAP-1 located on the vascular endothelial cell surface can act on circulating primary monoamines with the following reaction pathway.

RNH₂ + O₂ + H₂O → RCHO + H₂O₂ + NH₃

The physiological substrates of VAP-1 SSAO in man have not been clearly identified. However, methylamine is a good substrate for VAP-1 SSAO. Methylamine is a product of various human biochemical pathways for the degradation of creatinine, sarcosine and adrenaline, and is found in various mammalian tissues and in blood. It can also be derived from the diet by gut bacterial degradation of dietary precursors. The concentration of methylamine in the blood can be increased in certain physiological and pathological situations such as diabetes. Another potential physiological substrate is aminoacetone.

VAP-1 SSAO activity has been proposed to be directly involved in the pathway of leukocyte adhesion to endothelial cells by a novel mechanism involving direct interaction with an amine substrate presented on a VAP-1 ligand expressed on the surface of a leukocyte (Salmi et al. Immunity, vol. 14, pp. 265-276 (2001)). This publication describes the direct involvement of VAP-1 SSAO activity in the process of adhesion of leukocytes to endothelium. Thus inhibitors of VAP-1 SSAO activity could be expected to reduce leukocyte adhesion in areas of inflammation and thereby reduce leukocyte trafficking into the inflamed region and therefore the inflammatory process itself.

In human clinical tissue samples expression of VAP-1 is induced at sites of inflammation. This increased level of VAP-1 can lead to increased production of H₂O₂ generated from the action of the VAP-1 SSAO extracellular domain on monoamines present in the blood. This generation of H₂O₂ in the localized environment of the endothelial cell could initiate other cellular events. H₂O₂ is a known signaling molecule that can upregulate other adhesion molecules and this increased adhesion molecule expression may lead to enhanced leukocyte trafficking into areas in which VAP-1 is expressed. It also may be that other products of the VAP-1 SSAO reaction could have biological effects also contributing to the inflammatory process. Thus the products of the VAP-1 SSAO activity may be involved in an escalation of the inflammatory process which could be blocked by specific SSAO inhibitors.

VAP-1 SSAO may be involved in a number of other pathological conditions associated with an increased level of circulating amine substrates of VAP-1 SSAO. The oxidative deamination of these substrates would lead to an increase in the level of toxic aldehydes and oxygen radicals in the local environment of the endothelial cell which could damage the cells leading to vascular damage. It has been proposed that the vasculopathies such as retinopathy, neuropathy and nephropathy could be treated with specific inhibitors of SSAO activity.

Takahashi, H, et al., Yakugaku Zasshi 101(12):1154-1156 (1981) report the synthesis of a number of N-alkylaminoephedrines, including N-(isopropylideneamino)-ephedrine or R,S-(+)-(2-hydroxy-1-methyl-2-phenylethyl)methylhydrazone-2-propanone. These hydrazone compounds were synthesized to evaluate their effect on the bronchial musculature and were found not to exhibit any significant activity.

Grifantini, M., et al., Farmaco, Ed.Sci.23(3):197-203 (1968), report the synthesis of several alkyl- and acyl-derivatives of N-amino-1-ephedrine and N-amino-d-pseudoephedrine having antidepressant and monoamine oxidase inhibitory properties.

Jeffrey O'Sullivan et al., Biochimica et Biophysica Acta 1647 (2003) 367-371 report the inhibition of semicarbazide-sensitive amine oxidases by certain aminohexoses, namely glucosamine, galactosamine and mannosamine.

The international patent publications WO 02/020290 and WO 03/006003 disclose certain hydrazino compounds useful as specific VAP-1 SSAO inhibitors that modulate VAP-1 activity. These compounds are described as useful for the treatment of acute and chronic inflammatory conditions or diseases as well as diseases related to carbohydrate metabolism, aberrations in adipocyte differentiation or function and smooth muscle cell function, and various vascular diseases.

### OBJECTS AND SUMMARY OF THE INVENTION

Based on a recent study to be referred hereinafter, the inventors have found that transgenic mice overexpressing vascular adhesion protein-1 (VAP-1), chronically challenged with an additional SSAO substrate or an atherogenic diet, showed an increased glucose uptake, compared to non-transgenic mice. An end product of the SSAO metabolism, hydrogen peroxide, can produce insulin-like effects enhancing glucose uptake. Based on the findings, the inventors propose that amine oxidase enzyme activity is increased in metabolic syndrome as an attempt to regulate blood glucose levels, and that complications resulting from metabolic syndrome are subsequently promoted as a consequence of the enhanced enzyme activity.

According to one aspect, this invention concerns the use of an amine oxidase enzyme inhibitor for the manufacture of a pharmaceutical preparation useful in the treatment or prevention of metabolic syndrome and diseases or conditions resulting therefrom.

According to another aspect, the invention concerns the use of vitamin B1 or a metabolite thereof for the manufacture of a pharmaceutical preparation for use as an amine oxidase enzyme inhibitor in the treatment or prevention of metabolic syndrome and diseases or conditions resulting there from.

### DETAILED DESCRIPTION OF THE INVENTION

The term "metabolic syndrome" shall be understood to include the following abnormalities: central obesity, dyslipidemia including particularly hypertriglyceridemia, low HDL cholesterol, small dense LDL particles and postpranial lipemia; glucose intolerance such as impaired fasting glucose; insulin resistance and hypertension.

The term "complications related to metabolic syndrome" shall be understood to include any disease or disorder resulting from said syndrome. Such diseases are particularly different types of microalbuminuria; impaired fibrinolysis and increased coagulability including elevated PAI-1, elevated fibrinogen and increased levels of von Willebrand factor; signs of chronic inflammation such as elevated CRP; endothelial dysfunction such as impaired endothelium-dependent vasodilation; fatty liver disease and microangiopathy. As non-limiting examples of specific diseases and disorders can be mentioned atherosclerosis, vascular retinopathies, retinopathy, glomerulosclerosis, nephropathy, nephrotic syndrome, polyneuropathy, mononeuropathies, autonomic neuropathy, glaucoma, grey cataract, foot ulcers, joint problems, and increased risk of infection.

According to Bo Isomaa, Life Sciences 73 (2003) 2395-2411, a person is suffering from metabolic syndrome in case three of the following five conditions are fulfilled:
1. Fasting plasma glucose > 6.1 mmol/l
2. Hypertriglyceridemia; triglycerides > 1.7 mmol/l
3. Low HDL cholesterol; < 1.0 mmol/l (male) and < 1.3 mmol/l (female)
4. Hypertension; blood pressure > 130/85 and/or medication
5. Central obesity; waist circumference > 102 cm (male) and > 88 cm (female).

The term "prevention" shall be understood to include complete prevention, prophylaxis, as well as lowering the individual's risk of falling ill with said disease or condition. The term shall also be understood to include alleviation of symptoms already developed.

The tem "treatment" or "treating" shall be understood to include complete curing of a disease or condition, as well as amelioration or alleviation of said disease or condition.

The term "individual" refers to a human or animal subject.

The term "amine oxidase enzyme inhibitor" shall here be understood to cover any known or still undiscovered compound having this activity. It shall also be understood to cover any isomer, isomeric mixture, and any pharmaceutically or physiologically acceptable salt of such a compound.

The amine oxidase enzyme inhibitor is especially useful in the treatment or prevention of complications derived from metabolic syndromes.

When used in a pharmaceutical composition for use in prevention or treatment of metabolic syndrome and diseases or conditions resulting therefrom, the amine oxidase enzyme inhibitor, its isomer, isomer mixture or its pharmaceutically acceptable salt can be administered by various routes. For example, administration can be by parenteral, subcutaneous, intravenous, intraarticular, intrathecal, intramuscular, intraperitoneal, or intradermal injections, or by transdermal, buccal, oromucosal, ocular routes or via inhalation. Alternatively, or concurrently, administration can be by the oral route. Particularly preferred is oral administration. Suitable oral formulations include e.g. conventional or slow-release tablets and gelatine capsules.

The required dosage of the compounds will vary with the particular disease or condition being treated, the severity of the condition, the duration of the treatment, the administration route and the specific compound being employed.

Thus, a typical dose is in the dosage range of about 0.1 microgram/kg to about 300 mg/kg, preferably between 1.0 microgram/kg to 10 mg/kg body weight. Compounds of the present invention may be administered in a single daily dose, or the total daily dosage may be administered in divided doses of two, three or four times daily.

As examples of such inflammatory diseases or conditions can be mentioned
- connective tissue inflammatory diseases or conditions such as ankylosing spondylitis, Reiter's syndrome, psoriatic arthritis, osteoarthritis or degenerative joint disease, rheumatoid arthritis, Sjögren's syndrome, Bechet's syndrome, relapsing polychondritis, systemic lupus erythematosus, discoid lupus erythematosus, systemic sclerosis, eosinophilic fasciitis, polymyositis and dermatomyositis, polymyalgia rheumatica, vasculitis, temporal arteritis, polyarterisis nodosa, Wegner's granulamatosis, mixed connective tissue disease, and juvenile rheumatoid arthritis,
- gastrointestinal inflammatory diseases or conditions, such as Crohn's disease, ulcerative colitis, irritable bowel syndrome (spastic colon), fibrotic conditions of the liver, inflammation of the oral mucosa (stomatitis), and recurrent aphtous stomatitis,
- central nervous system inflammatory diseases or conditions, such as multiple sclerosis, Alzheimer's disease, and ischemia-reperfusion injury associated with ischemic stroke,
- pulmonary inflammatory diseases or conditions, such as asthma, chronic obstructive pulmonary disease, or adult respiratory distress syndrome,
- skin inflammatory diseases or conditions, such as contact dermatitis, atopic dermatitis, psoriasis, pityriasis rosea, lichen planus, and pityriasis rubra pilaris,
- inflammatory conditions related to tissue trauma or resulting from organ transplantations or other surgical operations.

As examples of diseases relating to aberrations in adipocyte differentiation or function or smooth muscle cell function can be mentioned atherosclerosis and obesity.

As examples of vascular diseases can be mentioned atheromatous ateriosclerosis, nonatheromateous ateriosclerosis, ischemic heart disease, peripheral aterial occlusion, thromboangiitis obliterans (Buerger's disease), or Raynaud's disease and phenomenon.

The invention will be illuminated by the following non-restrictive Experimental Section.

### EXPERIMENTAL SECTION

### SPECIFIC AIMS

This work investigates the in vivo significance of elevated semicarbazide sensitive amine oxidation (SSAO). To investigate the insulin mimicking capacity of VAP-1 activity and to test its ability to cause and/or exacerbate vascular complications we overexpressed VAP-1, an endothelial cell surface and soluble molecule possessing SSAO activity, in transgenic mice and then chronically challenged the mice for 15 months with additional SSAO substrate or an atherogenic diet.

### PRINCIPLE FINDINGS

### 1) Chronic human VAP-1 overexpression promotes obesity.

Despite a decreased caloric intake the transgenic mice had increased weight, body mass index (BMI), and subcutaneous abdominal and epididymal white adipose tissue (WAT) deposits when compared to controls.

### 2) Blood glucose is regulated by VAP-1 activity.

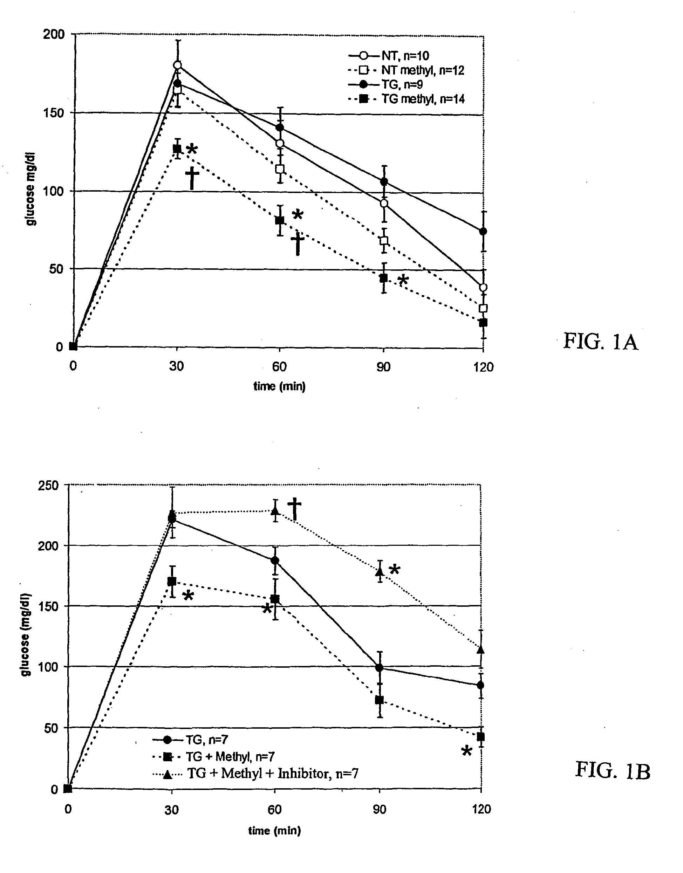

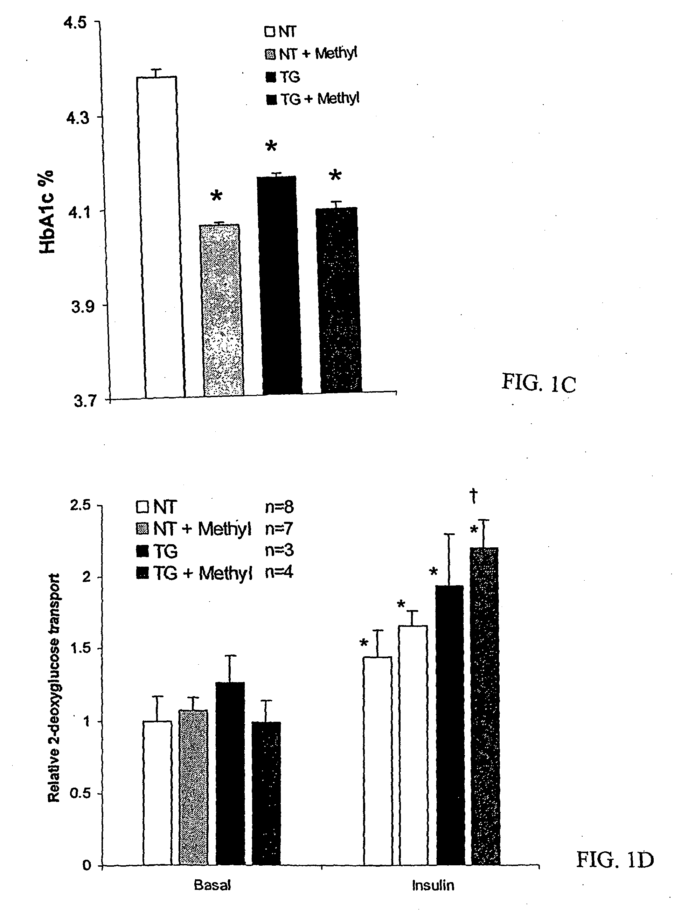

The combination of the transgene and methylamine enhanced glucose uptake when the mice were fasted and challenged with glucose (Figure 1A). This increase could be blocked with a small molecule inhibitor of SSAO (Figure 1B). The fasting glucose levels were also decreased by the transgene and HbA1c levels were decreased by the transgene and/or methylamine supplementation (Figure 1C). This amelioration of plasma glucose and glucose tolerance can be explained, at least in part, by improved insulin responsiveness of the skeletal muscle (Figure 1D).

### 3) VAP-1 overexpression increases advanced glycation end product (AGE) formation.

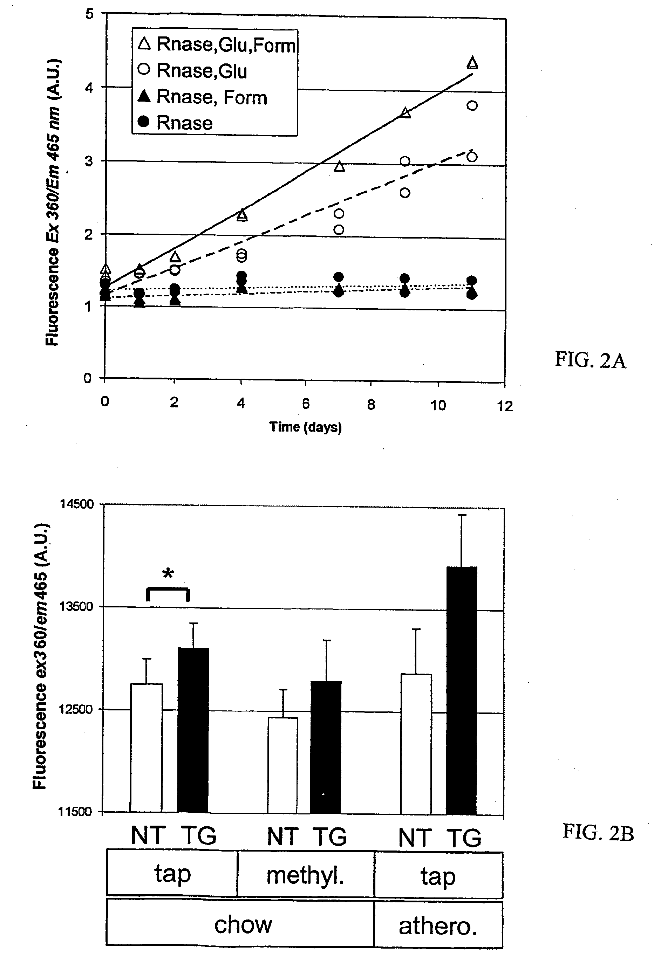

AGE-peptide levels were measured by fluorescence spectroscopy in the sera of the 64 week old mice. The level of AGE-specific fluorescence (*ex* 360, *em* 465) was increased in the presence of the transgene (Figure 2). The fluorescence from tryptophan (*ex* 280, *em* 333), an internal standard of protein concentration, did not significantly vary between the groups.

### 4) Methylamine supplementation promotes hypertension.

Systolic arterial blood pressure and heart rate were measured in all of the groups using the tail cuff method. Methylamine supplementation significantly elevated blood pressure but did not affect heart rate. A trend for elevated blood pressure was also found with the transgene alone.

### 5) Methylamine supplementation and the mTIEhVAP-1 transgene modify the progression of atherosclerosis.

The percentage of aorta surface area positive for Oil Red O staining was determined for the mice in each group. Methylamine supplementation increased the percentage of lesion formation when compared to untreated controls. The transgene decreased the number of lesions found in each aorta while leaving the total area of lesion formation unchanged suggesting that individual lesions are larger.

### 6) The mTIEhVAP-1 transgene inhibits renal and glomerular hypertrophy while at the same time promoting glomerulosclerosis.

Glomeruloscerosis is the main renal lesion in human and experimental diabetes. Its pathogenesis is controversial and the role of renal and glomerular hypertrophy in the pathogenesis of glomeruloscerosis is unclear. In this study, the two renal pathologies were uncoupled.

Total kidney mass was decreased by the transgene. An atherogenic diet induced increase in kidney mass was also inhibited by the transgene. Morphometric analysis further revealed a significant transgene specific reduction of total glomeruli surface areas and an inhibition of atherogenic diet induced glomerular hypertrophy.

In contrast, histological analysis of kidney sections revealed glomerulosclerosis (decreased capillary space, increased cell number, increased extracellular matrix, and a thicker glomerular stalk) in transgenic mice fed the atherogenic diet. Mild glomerular lesions were also found in non-transgenic mice fed the atherogenic diet but to a lesser extent than in the transgenic mice. In addition, occasional glomerular cysts were present in the transgenic mice fed the atherogenic diet but were not present in the similarly fed non-transgenic mice.

### CONCLUSIONS AND SIGNIFICANCE

Previous work has shown that SSAO activity is elevated in the serum of patients with congestive heart failure and specific inflammatory liver diseases. In this comprehensive study we for the first time show in vivo data indicating that this elevation of SSAO is not just a benign byproduct of the various disease states but that it is a cause of physiological and pathological changes. By performing these experiments in transgenic mice we reveal the consequences of increased SSAO without the additional complications of the disease states in which elevated SSAO is normally found.

We suggest that these changes result from the end products of SSAO substrate metabolism: hydrogen peroxide, ammonia, and aldehyde. On the one hand hydrogen peroxide can produce insulin-like effects that may be important for both obesity (lipogenesis promotion and lipolysis inhibition) and metabolic syndrome (increased glucose uptake) and on the other hand hydrogen peroxide is a key reactive oxygen species (ROS) that is implicated in endothelial cell toxicity and cardiovascular pathology. In addition, ammonia and formaldehyde are extremely reactive and cytotoxic chemicals that can promote aberrant, non-enzymatic glycation of proteins and may either directly or subsequently contribute to the late complications of diabetes such as hypertension, atherosclerosis and nephropathy.

Although these findings appear diametrically opposed they are not mutually exclusive. We propose that VAP-1 activity may be increased in metabolic syndrome as an attempt to regulate blood glucose levels and that vascular damage is subsequently promoted as a secondary consequence of this chronic enzyme activity. This suggests that manipulation of VAP-1 has potential to serve as a therapeutic treatment in conditions related to metabolic syndrome.

### EXPERIMENTS:

### Reference to Figures 1A-1D:

Human VAP-1 over-expression and methylamine supplementation enhance glucose tolerance. The increases in blood glucose over fasting blood glucose levels at 30, 60, 90, and 120 minutes after glucose challenge (2.0 g IP glucose/kg body weight) are expressed as mean ± SEM. (A) Glucose challenge of 26 week old non-transgenic (open symbols) and mTIEhVAP-1 transgenic mice (filled symbols) with normal tap water (solid lines) or with methylamine supplemented water (dashed lines). Significant effects on the rate of glucose clearance were identified by comparing the areas under the curve (AUC) of individual mice with an ANOVA (methylamine p=0.0002 and methylamine/transgene interaction p=0.046) and by comparing glucose levels at individual time points with unpaired Student's t-tests (asterisks, p<0.01 versus non-transgenic mice with normal water; crosses, p<0.02 versus non-transgenic mice with methylamine). (B) Glucose challenge of 10-12 week old transgenic mice with normal tap water (solid line) followed by 16 days of treatment with methylamine supplemented water (squares and dashed lines), and methylamine supplemented water as well as SSAO inhibitor injections (triangles and dashed lines). Significant effects were identified with paired Student's t-tests at individual time points (asterisks, p<0.01 and cross, p=0.037 versus transgenic mice with normal water) and for the AUC (normal water vs. methylamine treated, p=0.006; methylamine treated vs. methylamine treated plus SSAO inhibitor, p=0.0005; normal water vs. methylamine treated plus SSAO inhibitor, p=0.0008). (C) The mean ± SEM percent of glycosylated hemoglobin (HbA1c) was determined from the blood of fasted, 57-59 week old non-transgenic (white bars) and transgenic (black bars) mice with (gray strips) or without oral methylamine supplementation. Significant effects on HbAlc levels were found with a mixed model ANOVA (transgene p=0.03 and methylamine supplementation p<0.0001), and a transgene/methylamine interaction was identified (p=0.01). Asterisks, unpaired students t-tests, p<0.01 verses non-transgenic mice with normal food and drink. (D) Glucose transport activity of muscles from non-transgenic and mTIEhVAP-1 transgenic mice. The soleus muscles from non-transgenic (NT, white bars) and transgenic (TG, black bars) mice with (gray strips) or without oral methylamine supplementation for 14 days were used for measurement of basal and insulin-stimulated rates of 2-[1-¹⁴C]-deoxy-D-glucose transport. Values are represented as the mean ± SEM of the fold increase in transport compared to basal transport in non-transgenic mice with no treatment. Asterisks, p<0.05 vs. basal non-transgenic samples. Cross, significant difference when compared to non-transgenic mice with insulin alone (p=0.02) or non-transgenic mice with insulin plus methylamine (p=0.01).

### Reference to Figures 2A-2B:

(A) The SSAO reaction product, formaldehyde, promotes AGE formation in vitro. The AGE- specific fluorescence spectra (*ex* 360/*em* 465) of protein samples (20 mg/ml RNase) incubated at 37°C with or without 0.5 M glucose and 0.008% formaldehyde was periodically measured for 11 days. AU, arbitrary units. (B) The mTIEhVAP-1 transgene increases AGE-peptide levels in the sera of 64 week old mice. The AGE- specific fluorescence spectra (*ex* 360/*em* 465) of non-transgenic (NT, open bars) and transgenic (TG, solid bars) mice after chronic feeding with normal food (chow) and water (tap), methylamine supplemented water (Methyl.), or an atherogenic diet (athero.). A significant increase in AGE- specific fluorescence was found with the transgene when analyzing all of the treatments together (ANOVA, transgene effect p=0.05) and when analyzing the mice with just normal food and water (asterisk, unpaired students t-test, p=0.05). AU, arbitrary units.

We propose that SSAO activity is increased in diabetes as an attempt to regulate blood glucose levels and that vascular complications are subsequently promoted as a secondary consequence of chronic SSAO activity. When the in vivo levels of VAP-1 are increased (in response to diabetes or by transgenic overexpression) the potential for SSAO activity is increased. The deamination of primary amines, such as methylamine, by VAP-1 produces the biologically active compounds hydrogen peroxide, ammonia, and aldehyde. These compounds in turn can produce beneficial insulin-like effects while at the same time promoting AGE formation and vascular damage that is typical of diabetes.

### Vitamin B1 and its metabolites as VAP-1 inhibitors:

Fluorometric Detection of SSAO-mediated H₂O₂ Formation:

SSAO activity of the cells was measured using Amplex Red reagent (10-acetyl-3,7-dihydroxyphenoxazine; Molecular Probes Europe BV), a highly sensitive and stable probe for H₂O₂. Cultured cells (VAP-1 transfected CHO cells and their mock transfected controls) were rinsed with Krebs Ringer phosphate glucose (KRPG; 145 mM NaCl, 5.7 mM sodium phosphate, 4.86 mM KCI, 0.54 mM CaCl₂, 1.22 mM MgSO₄, 5.5 mM glucose, pH 7.35) and pre-incubated 30 min at 37°C in 200 µl KRPG containing thiamine, penicillin or ampicillin (1mg/ml). Catalytic reaction was initiated by addition of benzylamine as substrates and H₂O₂-detecting mixture containing horseradish peroxidase (final concentration 0.8 U/ml) and Amplex Red reagent (60 µM). The plates were incubated for 1-2 hours at 37°C in the final volume of 250 µl, the bathing medium was clarified by centrifugation and placed in aliquots (200 µl) into white non-phosphorescent microplates (Cliniplate). Fluorescence intensity of the samples was measured (excitation, 545 nm; emission, 590 nm; Tecan ULTRA fluoropolarometer) and H₂O₂ concentration was calculated from calibration curves generated by serial dilutions of either standard H₂O₂ or resorufin, the product of the Amplex Red reaction (Molecular Probes).

### Radiochemical Measurements of Monoamine Oxidase Activity

Amine oxidase activity was assayed radiochemically using [7-¹⁴C]-benzylamine hydrochloride (spec. act. 57 mCi/mmol, Amersham) as a substrate and 25 microliters of serum. The reaction was initiated by addition of 6 µmol/L [¹⁴C]-benzylamine (40000 dpm) and terminated after 1 hour by citric acid. The aldehydes were extracted into toluene containing diphenyloxazole and the formation of [¹⁴C]-labelled benzaldehyde was quantified by scintillation counting. Potential inhibition of serum VAP-1/SSAO enzymatic activity was measured in presence of indicated concentrations of thiamine. Finally, enzymatic activity of serum VAP-1/SSAO of six volunteers was measured before and after eating vitamin B1 100 mg/day for 5 to 9 days.

### Results

The inhibitory percentage obtained with thiamine after treating VAP-1 transfected CHO cells was 37 % with 1mg/ml and 38 % with 200 micrograms/ml and 7% with 100 micrograms/ml in fluorometric assay, whereas controls, penicillin and ampicillin, did not inhibit the activity. In vitro thiamine decreased serum VAP-1/SSAO activity as follows: 1 mg/ml , 87%; 800 micrograms/ml, 70%; 400 micrograms/ml 39%; 200 micrograms/ml 32%. In vivo response of VAP-1/SSAO enzymatic activity to vitamin B1 varied between individuals. Four of the six volunteers responded to vitamin B. Their VAP-1 enzyme activity at the end of the experiment had decreased: 25, 2, 32 and 16 % (mean 19%).

## Claims

1. The use of an amine oxidase enzyme inhibitor for the manufacture of a pharmaceutical preparation for use in the treatment or prevention of metabolic syndrome and diseases or conditions resulting therefrom.

2. The use according to claim 1 wherein the disease is a complication of a metabolic syndrome.

3. The use according to claim 2, wherein the complication is microalbuminuria; impaired fibrinolysis and increased coagulability including elevated PAI-1, elevated fibrinogen and increased levels of von Willebrand factor; signs of chronic inflammation such as elevated CRP; endothelial dysfunction such as impaired endothelium-dependent vasodilation; fatty liver disease and microangiopathy or any condition or disease derived thereof.

4. The use according to claim 2 or 3, wherein the complication resulting from metabolic syndrome is selected from the group consisting of atherosclerosis, vascular retinopathies, retinopathy, glomerulosclerosis, nephropathy, nephrotic syndrome, polyneuropathy, mononeuropathies, autonomic neuropathy, glaucoma, grey cataract, foot ulcers, joint problems, and increased risk of infection.

5. The use of vitamin B1 or a metabolite thereof for the manufacture of a pharmaceutical preparation for use as an amine oxidase enzyme inhibitor in the treatment or prevention of metabolic syndrome and diseases or conditions resulting therefrom.

## Patentansprüche

1. Verwendung eines Aminoxidase-Enzym-Inhibitors für die Herstellung einer pharmazeutischen Zubereitung zur Verwendung bei der Behandlung oder Prävention von metabolischem Syndrom und Erkrankungen oder Zuständen, die daraus resultieren.

2. Verwendung gemäß Anspruch 1, wobei die Erkrankung eine Komplikation eines metabolischen Syndroms ist.

3. Verwendung gemäß Anspruch 2, wobei die Komplikation Mikroalbuminurie; beeinträchtigte Fibrinolyse und erhöhte Gerinnungsfähigkeit, einschließlich erhöhtem PAI-1, erhöhtem Fibrinogen und erhöhten Spiegeln an von-Willebrand-Faktor; Anzeichen chronischer Entzündung, zum Beispiel erhöhtes CRP; Endothelial-Dysfunktion, zum Beispiel verschlechterte Endothel-abhängige Vasodilatation; Fettlebererkrankung und Mikroangiopathie oder ein beliebiger daraus herrührender Zustand oder eine beliebige daraus herrührende Erkrankung ist.

4. Verwendung gemäß Anspruch 2 oder 3, wobei die Komplikation, die aus metabolischem Syndrom resultiert, ausgewählt ist aus der Gruppe, bestehend aus Atherosklerose, vaskulären Retinopathien, Retinopathie, Glomerulosklerose, Nephropathie, nephrotischem Syndrom, Polyneuropathie, Mononeuropathien, autonomer Neuropathie, Glaukom, grauem Star, Ulzera des Fußes, Gelenkproblemen und erhöhtem Infektionsrisiko.

5. Verwendung von Vitamin B1 oder einem Metaboliten davon für die Herstellung einer pharmazeutischen Zubereitung zur Verwendung als ein Aminoxidase-Enzym-Inhibitor bei der Behandlung oder Prävention von metabolischem Syndrom und Erkrankungen oder Zuständen, die daraus resultieren.

## Revendications

1. Utilisation d'un inhibiteur d'enzyme amine-oxydase pour la fabrication d'une préparation pharmaceutique à utiliser dans le traitement ou la prévention du syndrome métabolique et des maladies ou affections résultant de celui-ci.

2. Utilisation selon la revendication 1, dans laquelle la maladie est une complication d'un syndrome métabolique.

3. Utilisation selon la revendication 2, dans laquelle la complication est une microalbuminurie ; une altération de la fibrinolyse et une hypercoagulabilité, comprenant un haut niveau de PAI-1, un haut niveau de fibrinogène et une forte concentration en facteur de von Willebrand ; les signes d'une inflammation chronique tels qu'un haut niveau de CRP ; un dysfonctionnement endothélial tel qu'une altération de la vasodilatation endothélium-dépendante ; une stéatose hépatique et une microangiopathie, ou toute maladie ou affection dérivée de ces complications.

4. Utilisation selon la revendication 2 ou 3, dans laquelle la complication résultant d'un syndrome métabolique est choisie dans le groupe formé par l'athérosclérose, les rétinopathies vasculaires, la rétinopathie, la glomérulosclérose, la néphropathie, le syndrome néphrotique, la polyneuropathie, les mononeuropathies, la neuropathie autonome, le glaucome, la cataracte grise, les ulcères des pieds, les problèmes articulaires et le risque accru d'infection.

5. Utilisation de vitamine B1 ou d'un métabolite de celle-ci pour la fabrication d'une préparation pharmaceutique à utiliser en tant qu'inhibiteur d'enzyme amine-oxydase dans le traitement ou la prévention du syndrome métabolique et des maladies ou affections résultant de celui-ci.
